# EUROPEAN PATENT SPECIFICATION

(11) **EP 1 387 822 B1**
(45) Date of publication and mention of the grant of the patent: **15.06.2005**
(21) Application number: 02727585.8
(22) Date of filing: 30.04.2002
(51) Int. Cl.: C07C 51/09, C07C 59/72, C07C 45/46, C07C 67/42

(54) **2-[4-(2,2,-DIHALOCYCLOPROPYL)PHENOXY]-ALKANOIC ACIDS AND ESTERS THEREOF PRODUCTION PROCESS**
VERFAHREN ZUR HERSTELLUNG VON 2-[4-(2,2,-DIHALOCYCLOPROPYL)PHENOXY]-ALKANSÄUREN UND IHRE ESTERN
PROCEDE DE PRODUCTION D'ACIDES 2- 4-(2, 2-DIHALOCYCLOPROPYL)PHENOXY]-ALKANOIQUES ET D'ESTERS DE CES DERNIERS

(30) Priority: 08.05.2001 IT MI20010936
(43) Date of publication of application: 11.02.2004
(73) Proprietor: LABORATORIO CHIMICO INTERNAZIONALE S.p.A., 20122 Milano (IT)
(72) Inventor: VILLANI, Flavio, I-43100 Parma (IT); NARDI, Antonio, I-20037 Paderno Dugnano (IT); SALVI, Annibale, I-20131 Milano (IT); FALABELLA, Giovanna, I-20152 Milan (IT)
(74) Representative: Gervasi, Gemma, Dr.
(86) International application number: PCT/EP2002/004747
(87) International publication number: WO 2002/090307

(56) References cited:
- US-A- 3 948 973
- US-A- 4 739 101
- US-A- 5 142 093
- KUKINKOVICH, O.G.; TISHCHENKO, I.G.; REZNIKOV, I.V.; PAP, A.A.: "Electron-donating characteristics of halogenocyclopropyl groups. Kinetics of the solvolysis of substituted 2-phenyl-2-chloropropanes" JOURNAL OF ORGANIC CHEMISTRY OF THE USSR (EN), vol. 17, no. 3, 1981, pages 396-400, XP008007496
- KUSUYAMA, Y.; IKEDA, Y.: "The Estimation of the Electronic Effects of Cyclopropyl and 2,2-Dichlorocyclopropyl Groups" BULLETIN OF THE CHEMICAL SOCIETY OF JAPAN, vol. 46, no. 1, 1973, pages 204-209, XP002211884
- HORROM, B.W.; MAZDIYASNI, H.: "A Convenient preparation of 4.cyclopropylphenol" ORGANIC PREPARATIONS AND PROCEDURES INTERNATIONAL, vol. 24, no. 6, 1992, pages 696-698, XP008007483
- RILEY, P; HANZLIK, R.P.: "Electron transfer in P450 mechanisms. Microsomal metabolism of cyclopropylbenzene and p-cyclopropylanisole" XENOBIOTICA, vol. 24, no. 1, 1994, pages 1-16, XP008007497
- Juma'a R et al: Journ. Chem. Sco. Perkin Trans.; 1993, pp 43-47

## Description

### Field of the invention

The present invention refers to a 2-[4-(2,2-dihalocyclopropyl)phenoxy]-alkanoic acids and esters thereof synthesis process.

### Prior art

2-[4-(2,2-Dihalocyclopropyl)phenoxy]-alkanoic acids, and especially of 2-[4-(2,2-dichlorocyctopropyl)phenoxy]-2-methylpropanoic acid (Ciprofibrate) synthesis processes are well known in the art (cf. US patent 3,948,973 and the corresponding GB patent 1,385,828). Further routes of synthesis, characterised by overall yields of 35% to 45%, have been disclosed more recently (cf. EP 0 245 156 and EP 0 142 979).

Horrom et Al in "A convenient preparation of 4-cyclopropyl-phenol" Organic Preparation and Procedure International, vol.24, no.6, 1992 pages 696-698, report the preparation of the 4-cyclopropylphenol, namely the compound not having geminal halogen atoms by a Bayer Williger oxidation reaction of the corresponding 4- cyclopropyl-acetophenone and subsequent hydrolysis reaction by using lithium hydroxide in an aqueous alcoholic solvent.

US4739101 comprises the preparation of esters of formula (I) according to the following reaction scheme: 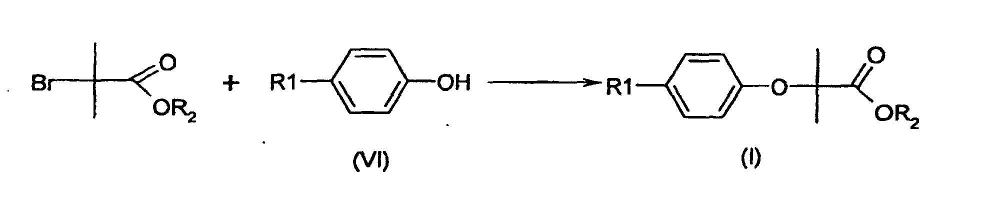 wherein R₁ is never a dihalocyclopropyl group.

Therefore, the need for new synthesis methods based on easy-to-do reactions giving high yields and high-purity intermediates, utilising commercially available reagents, and giving an overall yield - against the number of intermediate steps performed - higher to those achieved by the processes known in the art, is deeply felt.

### Summary of the invention

Now, it has been found a new synthesis process of 2-[4-(2,2-dihalocyclopropyl)phenoxy]-alkanoic acids and esters thereof overcoming the disadvantage of unsatisfactory overall yields typical of the processes of the prior art.

Surprisingly, the Applicant has found a new synthesis process including the Baeyer-Villiger reaction, to give 2-[4-(2,2-dihalocyclopropyl)phenoxy]-alkanoic acids and the corresponding linear or branched C₁-C₅ alkyl esters and in particular 2-[4-(2,2-dichlorocyclopropyl)phenoxy]-2-methylpropanoic acid (ciprofibrate) and the corresponding linear or branched C₁-C₅ alkyl esters.

The process object of the present invention gives the captioned product in 65% overall yield from dihalocyclopropyl benzene and in 56% overall yield from styrene (a raw material costing 10 to 15 times less than p-hydroxy acetophenone, which is used in the prior art).

Said yields are obtained with standard reagents, such as chloroform, NaOH, acetyl chloride, aluminium trichloride, hydrogen peroxide, maleic anhydride, potassium carbonate, sodium methylate--which are much cheaper than the respective raw materials used in the prior art-and with methods much simpler than those previously described.

### Brief description of the figures

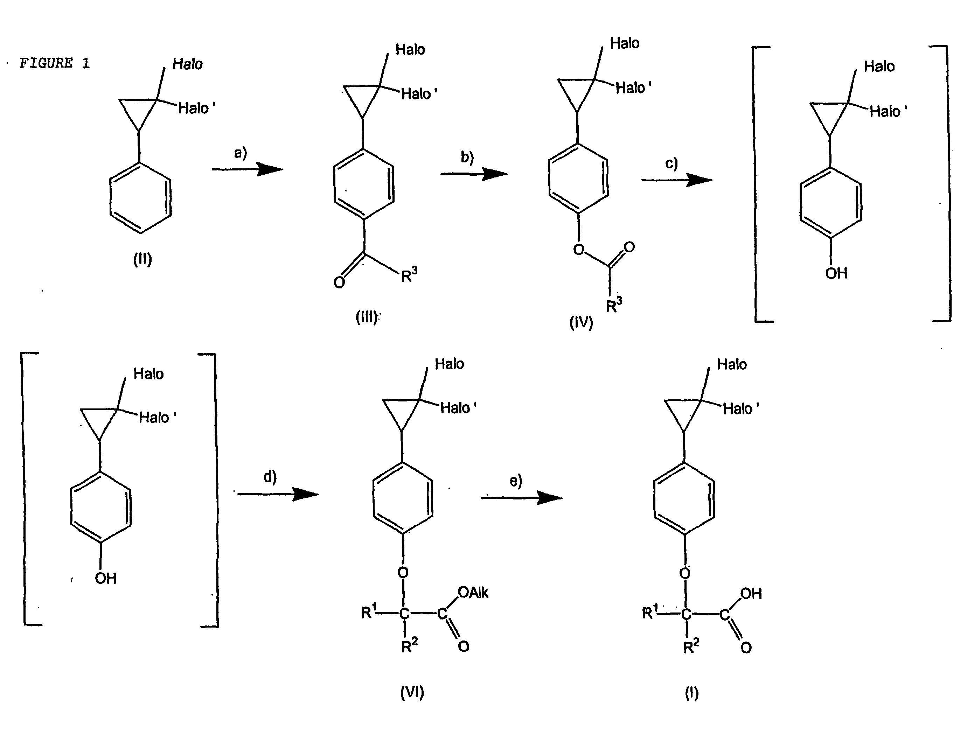

Figure 1: scheme of 2-[4-(2,2-dihalocyclopropyl)phenoxy]-alkanoic acids synthesis.

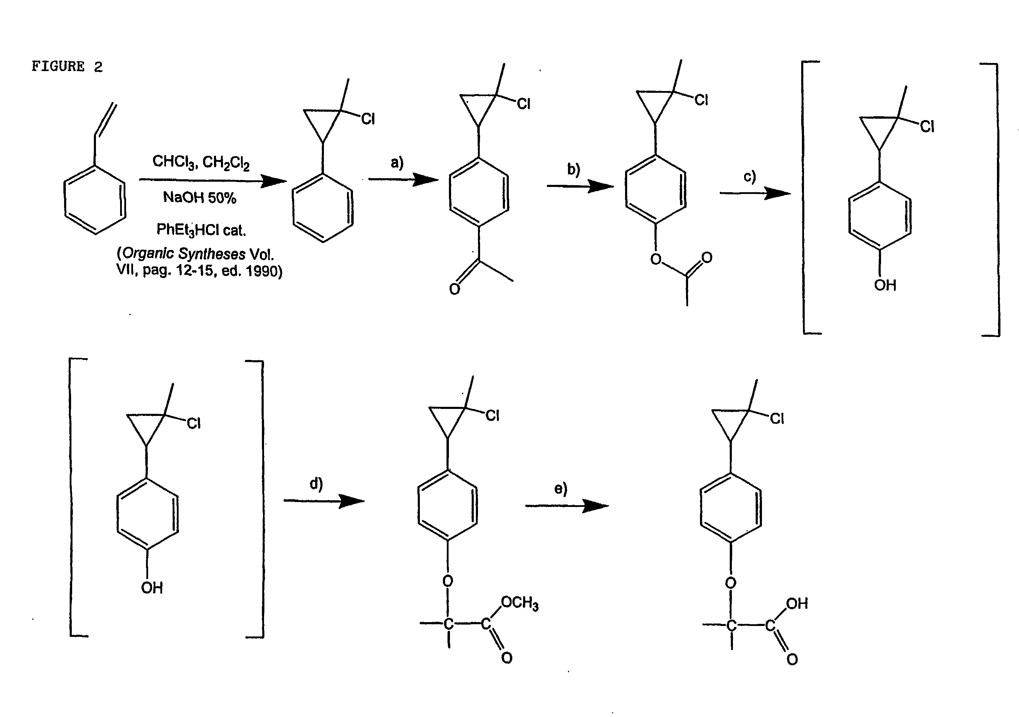

Figure 2: scheme of ciprofibrate synthesis.

### Detailed description of the invention

It is an object of the present invention to provide a process for the synthesis of 2-[4-(2,2-dihalocyclopropyl)phenoxy] alkanoic acids of general formula (I): 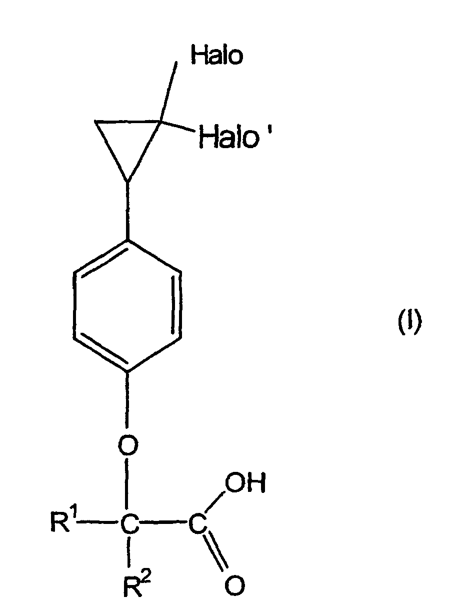 where Halo and Halo' are halogen atoms, which may be the same or different, R¹ and R² are C₁-C₃ alkyl groups which may be the same or different, said process comprising the following steps:
a) Friedel-Crafts reaction of the compound of general formula (II) 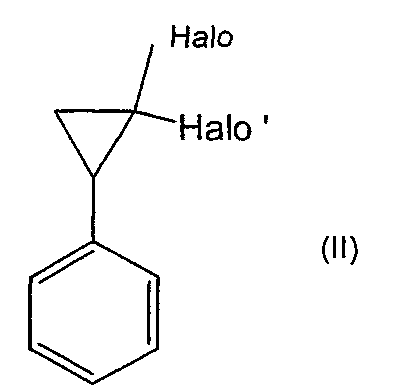 where Halo and Halo' have the above meanings, with an acyl halide: R³COX, where X is a halogen and R³ is a linear C₁-C₃ alkyl group, in the presence of a catalyst: Lewis acid, at a temperature ranging from -10°C to 50°C in halogenated aliphatic solvents or non-halogenated solvents;
b) Baeyer-Villiger oxidation reaction of the compound of general formula (III), obtained according to step a), 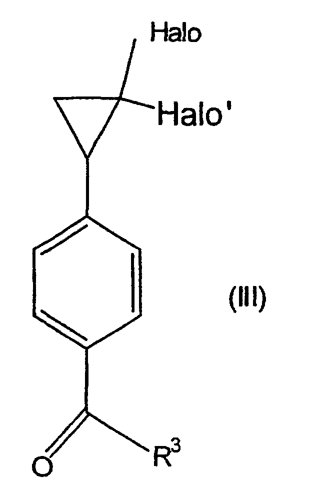 with peracids selected from the group consisting of peracetic acid, perbenzoic acid, trifluoroperacetic acid, 3,5-dinitroperbenzoic acid or with H₂O₂ and anhydrides selected from the group consisting of acetic anhydride, benzoic anhydride, trifluoroacetic anhydride, 3,5-dinitrobenzoic anhydride, phthalic anhydride, maleic anhydride, succinic anhydride or mixtures thereof, preferably the mixture of maleic anhydride and acetic anhydride; at a temperature ranging from -10°C to 80°C in halogenated aliphatic solvents or C₂-C₄ esters of C₂-C₄ aliphatic acids.
c) hydrolysis in an alcoholic solvent or in water-miscible ethers, with basic catalysis, at a temperature ranging from 10°C to 55°C, of the ester of general formula (IV), obtained according to step b): 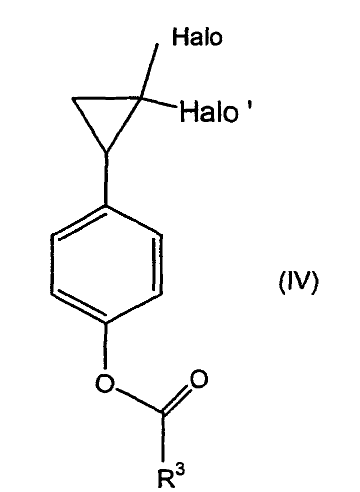 in which Halo, Halo' and R³ have the above meanings, to give the corresponding 4-dihalocyclopropyl-substituted phenol;
d) reaction of 4-dihalocyclopropyl-substituted phenol, obtained according to step c), with an α-haloester of general formula (V): (R¹)(R²)CX'COOAlk, where R¹, R² have the above meanings, Alk is a linear or branched C₁-C₅ alkyl group and X' is a halogen, in an alcoholic solvent in the presence of a base, to give the compound of formula (VI): 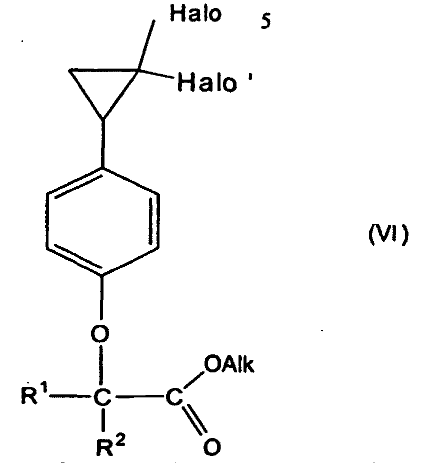
e) saponification of esters of general formula (VI), obtained according to step d), with alkaline metal hydroxides and acidification of the product prepared by saponification with acids to give the compound of general formula (I).
characterised in that:
i) in the hydrolysis of the compound of formula (IV), step c), the base is an inorganic base selected from the group consisting of alkaline carbonates, alkaline/alkaline earth dibasic borates, alkaline/alkaline earth dibasic phosphates or mixtures thereof and
ii) steps c), d) and e), take place in the same reaction vessel, without isolation of intermediates.

One of the preferred embodiments of the process object of the present invention includes, in addition to steps from a) to e) above said step f), i.e. the purification by crystallisation of the compound of general formula (I) by solubilisation in solvents selected from the group consisting of C₆-C₁₀ aromatic or aliphatic hydrocarbons and mixtures thereof.

According to the synthesis process of the present invention, in the Friedel-Crafts reaction, step a), the acyl halide is preferably acetyl chloride and the catalyst, Lewis acid, is selected from the group consisting of ferric chloride FeCl₃, zinc chloride ZnCl₂, tin chloride SnCl₂, aluminium chloride AlCl₃ or zeolites; more preferably, the acyl halide is acetyl chloride and the Lewis acid is aluminium chloride AlCl₃.

The Friedel-Crafts reaction, step a), is preferably carried out in methylene chloride, at a temperature ranging from -5°C to 30°C, and more preferably from 0°C to 20°C.

The Friedel-Crafts reaction, step a), is preferably carried out on compounds of general formula (II), in which Halo and Halo' are selected from the group consisting of chlorine and bromine, chlorine being especially preferred.

The Baeyer-Villiger reaction, step b), is preferably carried out in methylene chloride, at a temperature ranging from -5°C to 60°C, more preferably from 0 to 50°C.

In the hydrolysis of the compound of formula (IV), step c), the alcoholic solvent is preferably selected from the group consisting of linear or branched C₁-C₄ aliphatic alcohols or mixtures thereof. More preferably, the alcoholic solvent is selected from the group consisting of methanol, ethanol, n-propanol, isopropanol or mixtures thereof. Still more preferably, the alcoholic solvent is methanol and/or n-propanol. When the hydrolysis solvent is a water-miscible ether, it is selected out of the ethers well known in the field of the organic synthesis for the hydrolysis with basic catalysis of esters. The water-miscible ethers are preferably selected from the group consisting of tetrahydrofuran (THF), dioxane, ethylene glycol monomethyl ether (METHYCELLOSOLVE®), ethylene glycol monoethyl ether (ETHYLCELLOSOLVE®). In step (e) preferably, the inorganic base is selected from the group consisting of sodium carbonate (Na₂CO₃), potassium carbonate (K₂CO₃), or mixtures thereof, potassium carbonate being especially preferred.

The hydrolysis, step c), is preferably carried out at a temperature ranging from 20°C to 45°C, more preferably from 25° to 40°C.

In the α-haloester utilised in reaction step d) the alkyl groups R¹ and R², which may be the same or different, are selected from the group consisting of methyl, ethyl, n-propyl and isopropyl, the Alk substituent is preferably a linear or branched C₁-C₅ alkyl group, and more preferably is selected from the group consisting of methyl, ethyl, n-propyl, isopropyl, and X' is selected out of chlorine and bromine. In particular, the α-haloester can be easily found on the market and can be obtained from the corresponding α-haloacid.

In step d) the alcoholic solvent is preferably selected from the group consisting of linear or branched C₁-C₄ aliphatic alcohols or mixtures thereof. More preferably, the alcoholic solvent is selected from the group consisting of methanol, ethanol, n-propanol, isopropanol or mixtures thereof. Step d) utilises an inorganic base preferably selected from the group consisting of alkaline carbonates or mixtures thereof, or organic bases selected from the group consisting of C₁-C₄ alkaline alcoholates or mixtures thereof. More preferably, the inorganic base is selected from the group consisting of sodium carbonate (Na₂CO₃), potassium carbonate (K₂CO₃), or mixtures thereof, potassium carbonate being especially preferred; the organic base is selected from the group consisting of sodium methylate (CH₃ONa) and sodium ethylate (C₂H₅ONa) or mixtures thereof, sodium methylate being especially preferred. Still more preferably, the alcoholic solvent is isopropanol and the organic base is a solution of sodium methylate in methanol or the alcoholic solvent is n-propanol and the inorganic base is potassium carbonate.

In step e) the saponification preferably occurs with aqueous solutions of sodium carbonate or potassium carbonate and the acidification occurs with acids selected from the group consisting of sulphuric- or phosphoric- or acetic acid.

The purification by crystallisation of the compound of formula (I), step f), preferably utilises a solvent mixture of toluene and n-hexane, especially in a ratio equal to 1:4 by vol.

Out of the 2-[4-(2,2-dihalocyclopropyl)phenoxy]-alkanoic acids of general formula (I), and the corresponding alkyl esters of general formula (VI), particularly interesting are the 2-[4-(2,2-dihalocyclopropyl)phenoxy]-2-alkylpropanoic acids and the respective alkyl esters, in which the Alk group is a linear or branched C₁-C₅ alkyl, and especially the 2-[4-(2,2-dichlorocyclopropyl)phenoxy]-2-methylpropanoic acid (ciprofibrate) and the corresponding alkyl esters, in which the Alk group is a linear or branched C₁-C₅ alkyl.

A preferred embodiment of the synthesis process of 2-[4-(2,2-dihalocyclopropyl)phenoxy]-alkanoic acids of general formula (I), and of the corresponding alkyl esters of general formula (VI), as described above, is the synthesis process of 2-[4-(2,2-dihalocyclopropyl)phenoxy]-2-alkylpropanoic acids of formula (VII) 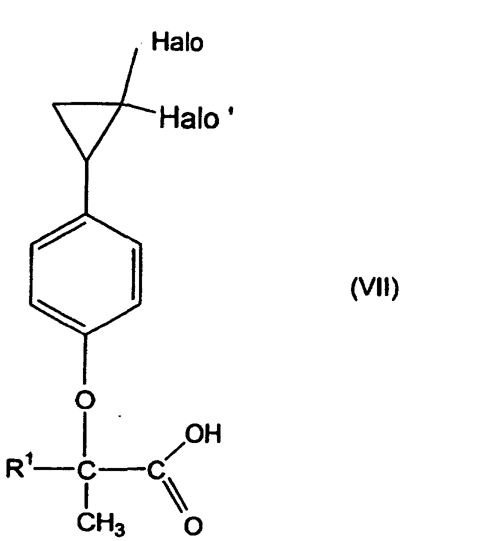 and of the corresponding linear or branched C₁-C₅ alkyl esters, where, in the α-haloester of general formula (V) utilised in reaction step d), one of the two alkyl groups, R¹ or R², is a methyl.

Another still more preferred embodiment of the synthesis process of 2-[4-(2,2-dihalocyclopropyl)phenoxy]-alkanoic acids of general formula (I) and of the corresponding esters of general formula (VI), as described above, is the synthesis process of 2-[4-(2,2-dichlorocyclopropyl)phenoxy]-2-methylpropanoic acid of formula (VIII) 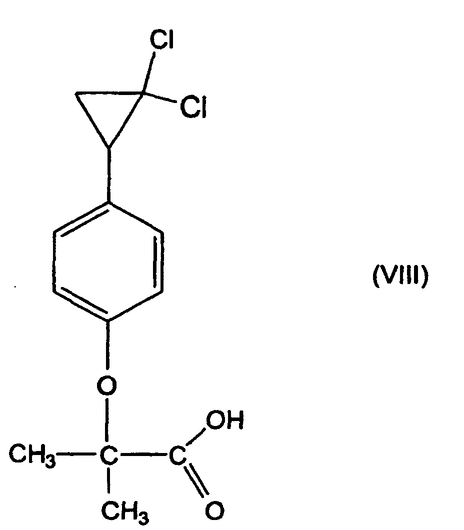 and of the corresponding linear or branched C₁-C₅ alkyl esters, in which the compound of general formula (II) is 2,2-dichlorocyclopropyl benzene and the α-haloester of general formula (V) is a linear or branched C₁-C₅ alkyl ester of α-bromoisobutyric acid, and more preferably is selected from the group consisting of the methyl-, ethyl-, n-propyl- and isopropyl ester of α-bromoisobutyric acid, and still more preferably is methyl α-bromoisobutyrate.

The 2-[4-(2,2-dihalocyclopropyl)phenoxy]-alkanoic acids of general formula (I) synthesis process, object of the present invention, involves the preparation―as reaction intermediates―of linear or branched C₁-C₅ alkyl esters of the corresponding acids; in particular the esters of general formula (VI), the linear or branched C₁-C₅ alkyl esters of 2-[4-(2,2-dihalocyclopropyl)phenoxy]-2-alkylpropanoic acids and the linear or branched C₁-C₅ alkyl esters, preferably the methyl ester, of 2-[4-(2,2-dichlorocyclopropyl)phenoxy]-2-methylpropanoic acid. For the synthesis of said esters, it is preferred that reaction steps c) and d) take place in the same reaction vessel, without isolation of intermediates.

The compounds of general formula (II), in which the Halo and Halo' substituents are preferably selected from the group consisting of chlorine and bromine, are commercially available, especially the 2,2-dichlorocyclopropyl benzene, which can be obtained as described in *Organic Syntheses, 7*, 12-15 (1990).

As proved by tests carried out by the Applicant, the claimed process gives 2-[4-(2,2-dihalocyclopropyl)phenoxy]-alkanoic acids of general formula (I)-in particular ciprofibrate-in overall yields in the order of 65% (63% for ciprofibrate), through a series of easy-to-do intermediate reactions, characterised by yields ranging from over 99% and over 75%, with commercially available solvents and reagents and under absolutely safe reaction conditions. Such a high overall yield was obtained *via* the Baeyer-Villiger reaction, which gives an isolable intermediate of general formula (IV), wherefrom the acid of general formula (I) is directly obtained, without the need to isolate p-dihalocyclopropyl phenol.

Reaction products and intermediates were characterised by ¹H-NMR, mass spectrometry and HPLC.

Some illustrative, but non-limiting examples of the present invention are described below.

### Example 1: Preparation of 4-(2,2-dichlorocyclopropyl) acetophenone from 2,2-dichlorocyclopropyl benzene

A 500 ml flask was fed with anhydrous AlCl₃ (40.4 g) and CH₂Cl₂ (141 ml). The resulting solution was cooled to 0°C and added dropwise slowly under stirring, with acetyl chloride (24.1 g), while the temperature was maintained at 20°C max. Stirring was continued at a temperature of 15°-20°C for 15 to 20 min. The solution was cooled to 0°-5°C and added dropwise and slowly with 2,2-dichlorocyclopropyl benzene (47.7 g), while the solution temperature was maintained below 10°C. Once the addition was completed, the solution was allowed to stir at a temperature of 15°-20°C for 3 hrs. The reaction was discontinued by pouring the solution so prepared into a 1-litre reactor containing 250 ml of cold water (0°-5°C). The lower organic phase was separated and washed with a mixture of water (235 ml) and methanol (47.5 ml). The organic phase was concentrated under vacuum to give 4-(2,2-dichlorocyclopropyl) acetophenone (58 g). Yield 99.3%; HPCL purity 98.5%.

The final product was characterised by ¹H-NMR and mass spectrometric analyses:
¹H-NMR (300 MHz, CDCl₃) (ppm): δ=1.92 (1H,dd); δ=2.03 (1H,dd); δ=2.6 (3H,s);
δ=2.94 (1H, dd); δ=7.34 (2H, m); δ=7.94 (2H,m).
Mass (El⁺) (m/e): 228 (molecular peak); 213-(-CH₃); 185-(-CH₃-C=O);

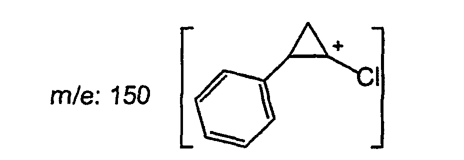

### Example 2: Preparation of 4-acetoxy-2,2-dichlorocyclopropyl benzene from 4-(2,2-dichlorocyclopropyl) acetophenone

A 1-litre flask was fed with CH₂Cl₂ (161 ml) and acetic anhydride (125.5 g). The solution was cooled to 0°-2°C and added dropwise with 40% w/v hydrogen peroxide, while the temperature was maintained at 5°C max.

The solution so prepared was allowed to stir at 5°-10°C for 1 hr and then added with maleic anhydride (93 g).

Stirring was continued for an additional hour while the temperature was allowed to rise. Then, the solution was cooled to 20°C and added dropwise with 4-(2,2-dichlorocyclopropyl)acetophenone (58 g).

When the reaction exothermicity tended to decrease, the solution was heated to reflux for a period of 6 hrs, then cooled to 20°C and added with water (160 ml).

After 20-min stirring, the phases that formed were separated: the organic phase was washed with water (120 ml) and then with a solution consisting of water (70 ml), 30% sodium hydroxide (18 ml) and sodium sulphite (9 g). The organic phase was separated and further washed with a solution consisting of water (120 ml), sodium sulphite (2.4 g) and again with water (120 ml). The organic phase separated at the end of the aforesaid washings, was dried, taken up with n-hexane (80 ml), heated to dissolution, at approx. 40°-45°C, allowed to crystallize, and cooled to 0°-5°C. The precipitate obtained was filtered and washed with cold hexane. After drying, 51 g of 4-acetoxy-2,2 dichloropropyl benzene was obtained.
Yield 82.2%.

The final product was characterised by ¹H-NMR, mass spectrometry and HPLC.
HPLC purity: 97.5%
¹H-NMR (300 MHz, CDCl₃) (ppm): δ=1.82 (1H,dd); δ=1.98(1H,dd); δ=2.29 (3H,s);
δ=2.88 (1 H, dd); δ=7.07 (2H, m); δ=7.25 (2H,m).
Mass (El⁺) (m/e): 244 (molecular peak); 202-(-CH₃-C=O);

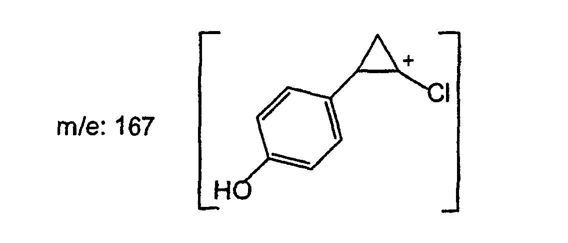

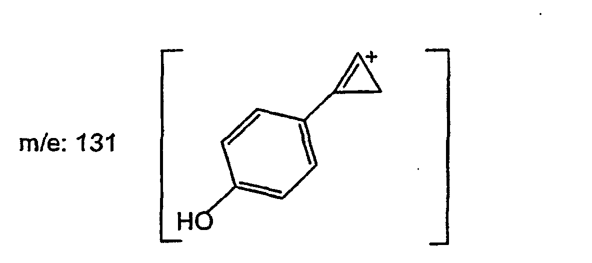

### Example 3: Preparation of 2-[4-(2,2-dichlorocyclopropyl)phenoxy]-2-methylpropanoic acid

A 500 ml flask was fed with 4-acetoxy-2,2-dichlorocyclopropyl benzene (49 g), methanol (80 ml) and K₂CO₃ (2 g). The resulting solution was allowed to stir at 35°-40°C for 3 hrs and distilled under vacuum at 40°-45°C for methanol elimination. The residue was taken up with isopropanol (160 ml) and methyl α-bromoisobutyrate (78.7 g). The reaction mixture was heated to reflux. The refluxing mixture was added dropwise, during 3 hrs, with a 30% (w/w) solution of sodium methylate in methanol (82 g). Once said addition was completed, the mixture was refluxed for 2 hrs, cooled to 50°C and added with a solution prepared by diluting 30% sodium hydroxide (71 g) in water (110 ml). The resulting reaction mixture was maintained at 50°C for 3 hrs, cooled, brought to pH 8.5 with diluted phosphoric acid, and concentrated under vacuum at 40°-45°C to a residue volume of approx. 220 ml. Said residue was added with water (180 ml) and toluene (80 ml); the phases were separated at 50°-55°C. The aqueous phase was rewashed according to the aforesaid procedure, and added with toluene (165 ml); the two-phase mass was acidified with 30% phosphoric acid and brought to pH 4.5. The aqueous phase was eliminated and the organic phase was concentrated under vacuum. The residue obtained was taken up with n-hexane (190 ml) and toluene (45 ml), heated to reflux, added with decolourising carbon (3 g), and clarified by hot filtration. 2-[4-(2,2-Dichlorocyclopropyl)phenoxy]-2-methylpropanoic acid (ciprofibrate) was crystallised from the clarified solution; crystallisation was completed by cooling to 0°C. 40 Grams of product was obtained after filtration, washing with hexane and drying. The concentration of mother liquors brought about the crystallisation of a second jet. After filtration and drying, it amounted to 5 g. The overall yield of the phases described above was 77.85%.

The final product was characterised by potentiometric titration, melting point measurement and HPLC.
Potentiometric titre 100.3%
HPLC purity 99.8%
m.p. 118.0°C

### Example 4: Preparation of 2-[4-(2,2-dichlorocyclopropyl)phenoxy]-2-methylpropanoic acid

A 1-litre flask was fed with 4-acetoxy-2,2-dichlorocyclopropyl benzene (35 g), methanol (60 ml) and K₂CO₃ (1.5 g). The resulting solution was allowed to stir at 35°-40°C for 3 hrs and distilled under vacuum at 40°-45°C for methanol elimination. The residue was taken up with n-propanol (100 ml) and added with K₂CO₃ (51 g) and methyl α-bromoisobutyrate (45 g). The reaction mixture was heated to reflux for 6 hrs, allowed to cool and added with water (130 ml). The phases were separated; the organic phase was caused to react with a solution prepared by diluting 30% sodium hydroxide (50 g) in water (80 ml). The resulting reaction mixture was maintained at 50°C for 1 hr, cooled, brought to pH 8.5 with diluted phosphoric acid, added with water (120 ml) and toluene (100 ml), and heated to 50°C. The phases were separated. The aqueous phase was rewashed according to the aforesaid procedure, and added with toluene (130 ml); the two-phase mass was acidified with 30% phosphoric acid and brought to pH 4.5. The aqueous phase was eliminated and the organic phase was concentrated under vacuum. The residue obtained was taken up with n-hexane (135 ml) and toluene (33 ml), heated to reflux, added with decolourising carbon (3 g), and clarified by hot filtration. 2-[4-(2,2-Dichiorocyclopropyl)phenoxy]-2-methylpropanoic acid (ciprofibrate) was crystallised from the clarified solution; crystallisation was completed by cooling to 0°C. 30 Grams of product was obtained after filtration, washing with hexane and drying. The concentration of mother liquors brought about the crystallisation of a second jet. After filtration and drying, it amounted to 2.2 g. The overall yield of the phases described above was 77.9%.

The final product was characterised by potentiometric titration, melting point measurement and HPLC.
Potentiometric titre 100.4%
HPLC purity 99.8%
m.p.117.6°C

## Claims

1. Process for the synthesis of 2-[4-(2,2-dihalocyclopropyl)phenoxy] alkanoic acids of general formula (I): 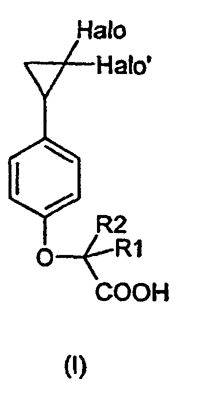 in which Halo and Halo' are halogen atoms, which are the same or different, R¹ and R² are C₁-C₃ alkyl groups, which are the same or different, said process comprising the following steps:
a) Friedel-Crafts reaction of the compound of general formula (II) 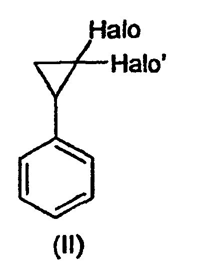 where Halo and Halo' have the above meanings, with an acyl halide: R³COX, where X is a halogen and R³ is a linear C₁-C₃ alkyl group, in the presence of a catalyst: Lewis acid, at a temperature ranging from -10°C to 50°C in halogenated aliphatic solvents or non-halogenated solvents;
b) Baeyer-Villiger oxidation reaction of the compound of general formula (III), 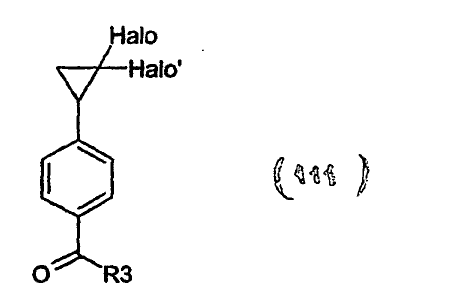 obtained according to step a), with peracids selected from the group consisting of peracetic acid, perbenzoic acid, trifluoroperacetic acid, 3,5-dinitroperbenzoic acid or with H₂O₂ and anhydrides selected from the group consisting of acetic anhydride, benzoic anhydride, trifluoroacetic anhydride, 3,5-dinitrobenzoic anhydride, phthalic anhydride, maleic anhydride, succinic anhydride or mixtures thereof, preferably the mixture of maleic anhydride and acetic anhydride; at a temperature ranging from -10°C to 80°C in halogenated aliphatic solvents or C₂-C₄ esters of C₂-C₄ aliphatic acids,
c) hydrolysis in an alcoholic solvent or in water-miscible ethers, with basic catalysis, at a temperature of 10°C to 55°C, of the ester of general formula (IV), obtained according to step b): 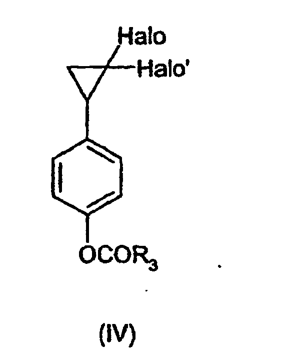 in which Halo, Halo' and R³ have the above meanings, to give the corresponding 4-dihalocyclopropyl-substituted phenol;
d) reaction of 4-dihalocyclopropyl-substituted phenol, obtained according to step c), with an α-haloester of general formula (V): (R¹)(R²)CX'CCOAlk, where R¹, R² have the above meanings, Alk is a linear or branched C₁-C₅ alkyl group and X' is a halogen, in an alcoholic solvent, with basic catalysis, to give the compound of formula (VI): 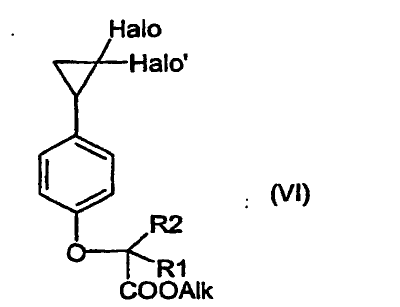
e) saponification of esters of general formula (VI), obtained according to step d), with alkaline metal hydroxides and acidification of the product prepared by saponification with acids to give the compound of general formula (I),
**characterised in that:**
i) in the hydrolysis of the compound of formula (IV), step c), the base is an inorganic base selected from the group consisting of alkaline carbonates, alkaline/alkaline earth dibasic borates, alkaline/alkaline earth dibasic phosphates or mixtures thereof and
ii) steps c), d) and e), take place in the same reaction vessel, without isolation of intermediates.

2. The process according to claim 1 further comprising step f) for purification by crystallisation of the compound of general formula (I) by solubilisation in solvents selected from the group consisting of C₆-C₁₀ aromatic or aliphatic hydrocarbons and mixtures thereof.

3. The process according to claim 2 wherein the solvents are a mixture of toluene and n-hexane.

4. The process according to claim 3 wherein the solvents are in a ratio equal to 1:4 by volume.

5. The process according to claim 1 wherein in the Friedel-Crafts reaction, step a), the acyl halide is acetyl chloride and the catalyst, Lewis acid, is selected from the group consisting of ferric chloride FeCl₃, zinc chloride ZnCl₂, tin chloride SnCl₂, aluminium chloride AlCl₃ or zeolites.

6. The process according to claim 5 wherein the acyl halide is acetyl chloride and the Lewis acid is aluminium chloride AlCl₃.

7. The process according to claim 1 wherein the Friedel-Crafts reaction, step a) is carried out in methylene chloride.

8. The process according to claim 1 wherein the Friedel-Crafts reaction, step a), is carried out at a temperature ranging from -5°C to 30°C, preferably from 0°C to 20 °C.

9. The process according to claim 1 wherein the Friedel-Crafts reaction, step a) is carried out on compounds of general formula (II), in which Halo and Halo' are selected from the group consisting of chlorine and bromine, optionally chlorine

10. The process according to claim 1, wherein the Baeyer-Villiger reaction, step b), is carried out in methylene chloride.

11. The process according to claim 1, wherein the Baeyer-Villiger reaction, step b), is carried out at a temperature ranging from -5°C to 60°C, optionally from 0 to 50°C.

12. The process according to claim 1 wherein in the hydrolysis of the compound of formula (IV), step c), the alcoholic solvent is selected from the group consisting of linear or branched C₁-C₄ aliphatic alcohols or mixtures thereof.

13. The process according to claim 12 wherein the alcoholic solvent is selected from the group consisting of methanol, ethanol, n-propanol, isopropanol or mixtures thereof.

14. The process according to claim 13 wherein the alcoholic solvent is methanol and/or n-propanol.

15. The process according to claim 1 wherein in the hydrolysis of the compound of formula (IV), step c), the solvent is a water-miscible ether.

16. The process according to claim 15 wherein the water-miscible ether is selected from the group consisting of tetrahydrofuran (THF), dioxane, ethylene glycol monomethyl ether (METHYCELLOSOLVE®), ethylene glycol monoethyl ether (ETHYLCELLOSOLVE®).

17. The process according to claim 1 wherein the inorganic base is selected from the group consisting of sodium carbonate (Na₂CO₃), potassium carbonate (K₂CO₃), or mixtures thereof.

18. The process according to claim 17 wherein the inorganic base is potassium carbonate.

19. The process according to claim 1 wherein the hydrolysis, step c), is carried out at a temperature ranging from 20°C to 45°C, optionally from 25 to 40°C.

20. The process according to claim 1 wherein in the α-haloester utilised in reaction step d) the alkyl groups R¹ and R², which are the same or different, are selected from the group consisting of methyl, ethyl, n-propyl and isopropyl.

21. The process according to claim 1 wherein in the α-haloester utilised in reaction step d) the Alk substituent is selected from the group consisting of methyl, ethyl, n-propyl, isopropyl.

22. The process according to claim 21 wherein the Alk substituent is a methyl.

23. The process according to claim 1 wherein in the α-haloester utilised in reaction step d) X' is selected out of chlorine and bromine.

24. The process according to claim 1 wherein in step d) the alcoholic solvent is selected from the group consisting of linear or branched C₁-C₄ aliphatic alcohols or mixtures thereof.

25. The process according to claim 24 wherein the alcoholic solvent is selected from the group consisting of methanol, ethanol, n-propanol, isopropanol or mixtures thereof.

26. The process according to Claim 1 wherein the base at step d) is an inorganic base selected from the group consisting of alkaline carbonates or mixtures thereof, or an organic base selected from the group consisting of C₁-C₄ alkaline alcoholates or mixtures thereof.

27. The process according to claim 26 wherein the inorganic base is selected from the group consisting of sodium carbonate (Na₂CO₃), potassium carbonate (K₂CO₃) or mixtures thereof.

28. The process according to claim 27 wherein the inorganic base is potassium carbonate.

29. The process according to claim 26 wherein the organic base is selected from the group consisting of sodium methylate (CH₃ONa) and sodium ethylate (C₂H₅ONa) or mixtures thereof.

30. The process according to claim 29 wherein the organic base is sodium methylate.

31. The process according to claim 1 wherein in step d) the alcoholic solvent is n-propanol and the base is potassium carbonate.

32. The process according to claim 1 wherein in step d) the alcoholic solvent is isopropanol and the base is a solution of sodium methylate in methanol.

33. The process according to claim 1 wherein the saponification, step e), occurs with aqueous solutions of sodium carbonate or potassium carbonate and the acidification occurs with acids selected from the group consisting of sulphuric-, phosphoric- or acetic acid.

34. The process according to any of claims 1 and 2 for preparing 2-[4-(2,2-dihalocyclopropyl)phenoxy]-2-alkylpropanoic acids of formula (VII) 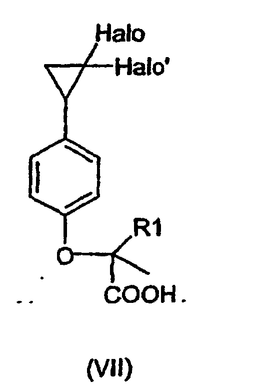 wherein in the α-haloester of general formula (V) utilised in reaction step d), one of the two alkyl groups, R¹ or R², is a methyl.

35. The process according to any of claims 1 and 2 for preparing the compound 2-[4-(2,2-dichlorocyclopropyl)phenoxy]-2-methylpropanoic acid of formula (VIII) 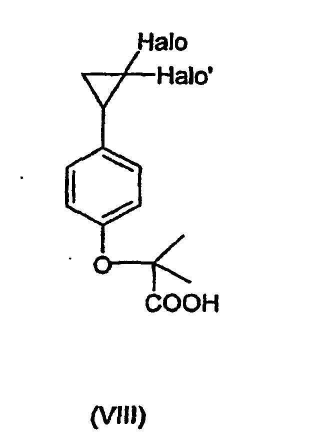 wherein the compound of general formula (II) is 2,2-dichlorocyclopropyl benzene and the α-haloester of general formula (V) is the linear or branched C₁-C₅ alkyl ester of α-bromoisobutyric acid.

36. The process according to claim 35 wherein the alkyl ester of α-bromoisobutyric acid is selected from the group consisting of methyl-, ethyl-, n-propyl- and isopropyl ester of α-bromoisobutyric acid.

37. The process according to claim 36 wherein the ester of α-bromoisobutyric acid is methyl α-bromoisobutyrate.

## Patentansprüche

1. Verfahren zur Herstellung von 2-[4-(2,2-Dihalocylcopropyl)phenoxy]alkansäuren mit der allgemeinen Formel (I): 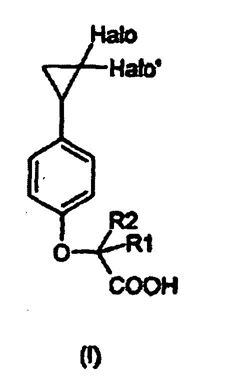 in welcher Halo und Halo' identische oder verschiedene Halogenatome und R¹ und R² identische oder verschiedene C₁-C₃-Alkylgruppen sind, wobei genanntes Verfahren folgende Schritte aufweist:
a) eine Friedel-Crafts-Reaktion der Verbindung mit der allgemeinen Formel (II) 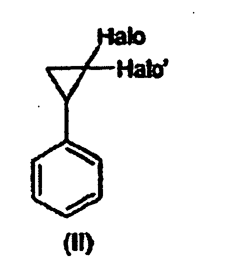 wobei Halo und Halo' die oben genannte Bedeutung haben, mit einem Säurehalogenid R³COX, wobei X ein Halogen ist und R³ eine lineare C₁-C₃-Alkylgruppe, in Gegenwart eines Katalysators, nämlich einer Lewissäure, bei einer Temperatur zwischen -10 °C und 50 °C in halogenierten aliphatischen Lösungsmitteln und nicht halogenierten Lösungsmitteln;
b) Baeyer-Villiger-Oxidation der Verbindung mit der allgemeinen Formel (III), 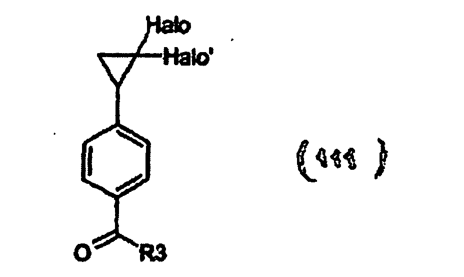 welche in Schritt a) erhalten wurde, mit Persäuren ausgewählt aus der Gruppe bestehend aus Peressigsäure, Perbenzoesäure, Trifluorperessigsäure, 3,5-Dinitroperbenzoesäure oder mit H₂O₂ und Anhydriden ausgewählt aus der Gruppe bestehend aus Essigsäureanhydrid, Benzoesäureanhydrid, Trifluoressigsäureanhydrid, 3,5-Dinitrobenzoesäureanhydrid, Phthalsäureanhydrid, Maleinsäureanhydrid, Bernsteinsäureanhydrid oder Mischungen daraus, vorzugsweise eine Mischung aus Maleinsäureanhydrid und Essigsäureanhydrid, bei einer Temperatur zwischen -10 °C und 80 °C in halogenierten aliphatischen Lösungsmitteln oder C₂-C₄-Estern von aliphatischen C₂-C₄-Säuren,
c) Hydrolyse in einem alkoholischen Lösungsmittel oder einem mit Wasser mischbaren Ether unter basischer Katalyse bei einer Temperatur zwischen 10 °C und 55 °C von Estern mit der allgemeinen Formel (IV), die durch Schritt b) erhalten wurden: 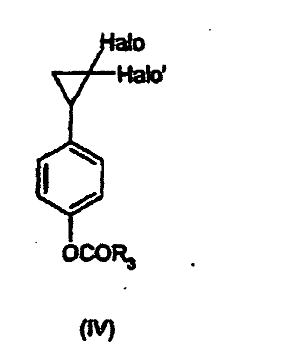 in welchen Halo, Halo' und R³ die oben genannten Bedeutungen haben, um das entsprechende 4-Dihalocyclopropyl-substituierte Phenol zu ergeben;
d) Reaktion des 4-Dihalocyclopropyl-substituierten Phenols, erhalten nach Schritt c), mit einem α-Haloester der allgemeinen Formel (V): (R1)(R2)CX'COOAlk, wobei R¹ und R² die oben genannte Bedeutung haben, Alk eine lineare oder verzweigte C₁-C₅-Alkylgruppe und X' ein Halogen ist, in einem alkoholischen Lösungsmittel unter basischer Katalyse, um die Verbindung mit der Formel (VI) zu ergeben: 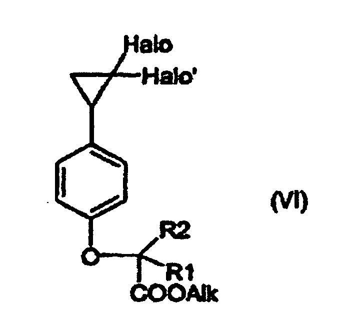
e) Verseifung der Ester mit der allgemeinen Formel (VI), erhalten nach Schritt d), mit Alkalimetallhydroxiden und Ansäuerung des durch die Verseifung hergestellten Produktes mit Säuren um die Verbindung mit der allgemeinen Formel (I) zu erhalten,
**dadurch gekennzeichnet, dass**:
i) bei der Hydrolyse der Verbindung mit der Formel (IV) in Schritt c) die Base eine anorganische Base ist, ausgewählt aus der Gruppe bestehend aus Alkalimetallcarbonaten, zweibasigen Alkalimetall- oder Erdalkalimetallboraten, Alkalimetall- oder Erdalkalimetallhydrogenphosphaten oder Mischungen daraus und
ii) die Schritte c), d) und e) im gleichen Reaktionsgefäß stattfinden, ohne Isolierung der Zwischenprodukte.

2. Verfahren nach Anspruch 1, weiterhin umfassend einen Schritt f) zur Reinigung durch Umkristallisierung der Verbindung der allgemeinen Formel (I) durch Lösen in Lösungsmitteln ausgewählt aus der Gruppe bestehend aus aromatischen oder aliphatischen C₆-C₁₀-Kohlenwasserstoffen und Mischungen daraus.

3. Verfahren nach Anspruch 2, wobei die Lösungsmittel eine Mischung aus Toluol und n-Hexan sind.

4. Verfahren nach Anspruch 3, wobei die Lösungsmittel in einem Volumenverhältnis von 1 : 4 vorliegen.

5. Verfahren nach Anspruch 1, wobei in der Friedel-Crafts-Reaktion in Schritt a) das Säurehalogenid ein Essigsäurechlorid und der Katalysator, die Lewissäure, ausgewählt wird aus der Gruppe bestehend aus Eisenchlorid FeCl₃, Zinkchlorid ZnCl, Zinnchlorid SnCl₂, Aluminiumchlorid AlCl₃ oder Zeolithen.

6. Verfahren nach Anspruch 5, wobei das Säurehalogenid Essigsäurechlorid und die Lewissäure Aluminiumchlorid AlCl₃ ist.

7. Verfahren nach Anspruch 1, wobei die Friedel-Crafts-Reaktion in Schritt a) in Methylenchlorid ausgeführt wird.

8. Verfahren nach Anspruch 1, wobei die Friedel-Crafts-Reaktion in Schritt a) in einem Temperaturbereich zwischen -5 °C und 30 °C, vorzugsweise zwischen 0 °C und 20 °C ausgeführt wird.

9. Verfahren nach Anspruch 1, wobei die Friedel-Crafts-Reaktion in Schritt a) an Verbindungen mit der allgemeinen Formel (II) ausgeführt wird, wobei Halo und Halo' ausgewählt werden aus der Gruppe bestehend aus Chlor und Brom, vorzugsweise Chlor:

10. Verfahren nach Anspruch 1, wobei die Baeyer-Villiger-Reaktion in Schritt b) in Methylenchlorid ausgeführt wird.

11. Verfahren nach Anspruch 1, wobei die Baeyer-Villiger-Reaktion in Schritt b) in einem Temperaturbereich zwischen -5 °C und 60 °C, vorzugsweise zwischen 0 °C und 50 °C ausgeführt wird.

12. Verfahren nach Anspruch 1, wobei bei der Hydrolyse der Verbindung mit der Formel (IV) in Schritt c) das alkoholische Lösungsmittel ausgewählt wird aus der Gruppe bestehend aus linearen oder verzweigten aliphatischen C₁-C₄-Alkoholen und Mischungen daraus.

13. Verfahren nach Anspruch 12, wobei das alkoholische Lösungsmittel ausgewählt wird aus der Gruppe bestehend aus Methanol, Ethanol, n-Propanol, Isopropanol oder Mischungen daraus.

14. Verfahren nach Anspruch 13, wobei das alkoholische Lösungsmittel Methanol und/oder n-Propanol ist.

15. Verfahren nach Anspruch 1, wobei bei der Hydrolyse der Verbindung mit der Formel (IV) in Schritt c) das Lösungsmittel ein mit Wasser mischbarer Ether ist.

16. Verfahren nach Anspruch 15, wobei der mit Wasser mischbare Ether ausgewählt wird aus der Gruppe bestehend aus Tetrahydrofuran (THF), Dioxan, Ethylenglykolmonomethylether (Methycellosolve®), Ethylenglykohnonoethylether (Ethylcellosolve®).

17. Verfahren nach Anspruch 1, wobei die anorganische Base ausgewählt wird aus der Gruppe bestehend aus Natriumcarbonat (Na₂CO₃), Kaliumcarbonat (K₂CO₃) oder Mischungen daraus.

18. Verfahren nach Anspruch 17, wobei die anorganische Base Kaliumcarbonat ist.

19. Verfahren nach Anspruch 1, wobei die Hydrolyse in Schritt c) bei einer Temperatur zwischen 20 °C und 45°C, vorzugsweise zwischen 25 und 40 °C ausgeführt wird.

20. Verfahren nach Anspruch 1, wobei bei dem in Reaktionsschritt d) verwendeten α-Haloester die Alkylgruppen R¹ und R², welche identisch oder verschieden sein können, ausgewählt werden aus der Gruppe bestehend aus Methyl-, Ethyl-, n-Propyl- und Isopropyl-.

21. Verfahren nach Anspruch 1, wobei bei dem in Reaktionsschritt d) verwendeten α-Haloester der Substituent Alk ausgewählt wird aus der Gruppe bestehend aus Methyl-, Ethyl-, n-Propyl- und Isopropyl-.

22. Verfahren nach Anspruch 21, wobei der Substituent Alk- Methyl- ist.

23. Verfahren nach Anspruch 1, wobei bei dem in Reaktionsschritt. d) verwendeten α-Haloester X' ausgewählt wird aus Chlor und Brom.

24. Verfahren nach Anspruch 1, wobei in Schritt d) das alkoholische Lösungsmittel ausgewählt wird aus der Gruppe bestehend aus linearen oder verzweigten aliphatischen C₁-C₄-Alkoholen oder Mischungen daraus.

25. Verfahren nach Anspruch 24, wobei das alkoholische Lösungsmittel ausgewählt wird aus der Gruppe bestehend aus Methanol, Ethanol, n-Propanol, Isopropanol oder Mischungen daraus.

26. Verfahren nach Anspruch 1, wobei die Base in Schritt d) eine anorganische Base, ausgewählt aus der Gruppe bestehend aus Alkalimetallcarbonaten oder Mischungen daraus, oder eine organische Base ist, ausgewählt aus der Gruppe bestehend aus C₁-C₄-Alkalimetallalkoholaten oder Mischungen daraus.

27. Verfahren nach Anspruch 26, wobei die anorganische Base ausgewählt wird aus der Gruppe bestehend aus Natriumcarbonat (Na₂CO₃), Kaliumcarbonat (K₂CO₃) oder Mischungen daraus.

28. Verfahren nach Anspruch 27, wobei die anorganische Base Kaliumcarbonat ist.

29. Verfahren nach Anspruch 26, wobei die organische Base ausgewählt wird aus der Gruppe bestehend aus Natriummethylat (CH₃ONa) und Natriumethylat (C₂H₅ONa) oder Mischungen daraus.

30. Verfahren nach Anspruch 30, wobei die organische Base Natriummethylat ist.

31. Verfahren nach Anspruch 1, wobei das alkoholische Lösungsmittel in Schritt d) n-Propanol und die Base Kaliumcarbonat ist.

32. Verfahren nach Anspruch 1, wobei das alkoholische Lösungsmittel in Schritt d) Isopropanol und die Base eine Lösung von Natriummethylat in Methanol ist.

33. Verfahren nach Anspruch 1, wobei die Verseifung in Schritt e) mit wässrigen Lösungen von Natriumcarbonat oder Kaliumcarbonat ausgeführt wird und das Ansäuern mit Säuren ausgewählt aus der Gruppe bestehend aus Schwefel-, Phosphor- oder Essigsäure ausgeführt wird.

34. Verfahren nach einem der Ansprüche 1 und 2 zur Herstellung von 2-[4-(2,2-Dihalocylcopropyl)phenoxy]-2-alkylpropionsäuren mit der Formel (VII): 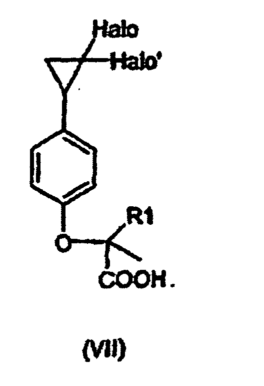 wobei bei dem in Reaktionsschritt d) verwendeten α-Haloester mit der allgemeinen Formel (V) eine der beiden Alkylgruppen R¹ oder R² eine Methylgruppe ist.

35. Verfahren nach einem der Ansprüche 1 und 2 zur Herstellung der Verbindung 2-[4-(2,2-Dihalocylcopropyl)phenoxy]-2-methylpropionsäure mit der Formel (VIII): 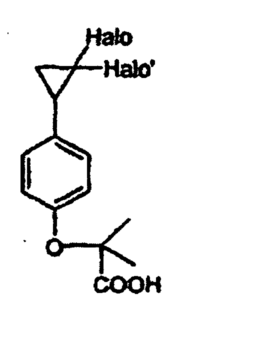 wobei die Verbindung mit der allgemeinen Formel (II) 2,2-Dichlorcyclopropylbenzol und der α-Haloester mit der allgemeinen Formel (V) ein linearer oder verzweigter C₁-C₅-Alkylester der α-Bromisobuttersäure ist.

36. Verfahren nach Anspruch 35, wobei der Alkylester der α-Bromisobuttersäure ausgewählt wird aus der Gruppe bestehend aus Methyl-, Ethyl-, n-Propyl- und Isopropylestem der α-Bromisobuttersäure.

37. Verfahren nach Anspruch 36, wobei der Ester der α-Bromisobuttersäure α-Bromisobuttersäuremethylester ist.

## Revendications

1. Procédé pour la synthèse d'acides 2-[4-(2,2-dihalocyclopropyl)phénoxy]alcanoïques de la formule générale (I): 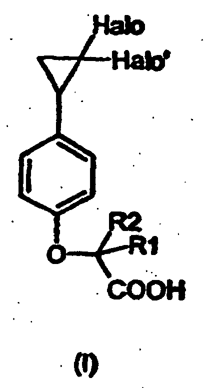 dans laquelle Halo, et Halo' sont des atomes d'halogène, qui sont identiques ou différents, R¹ et R² sont des groupes alkyles C₁-C₃, qui sont identiques ou différents, ledit procédé comprenant les étapes suivantes:
a) réaction de Friedel-Crafts du composé de formule générale (II) 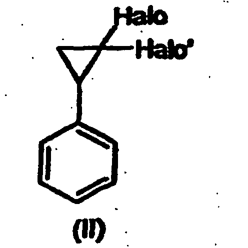 où Halo et Halo' ont les significations ci-dessus, avec un halogénure d'acyle: R³COX, où X est un halogène et R³ est un groupe alkyle C₁-C₃ linéaire, en présence d'un catalyseur: acide de Lewis, à une température allant de -10°C. à 50°C dans des solvants aliphatiques halogénés ou solvants non halogénés;
b) Réaction d'oxydation de Baeyer-Villiger du composé de formule générale (III), 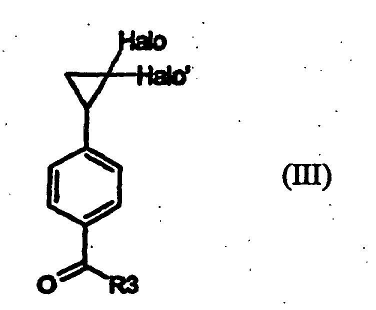 obtenu selon l'étape a), avec des peracides sélectionnés dans le groupe consistant en acide péracétique, acide perbenzoique, acide trifluoroperacétique, acide 3,5-dinitroperbenzoïque ou avec H₂O₂ et des anhydrides sélectionnés dans le groupe consistant en anhydride acétique, anhydride benzoïque, anhydride trifluoroacétique, anhydride 3,5-dinitrobenzoïque, anhydride succinique ou leurs mélanges, de préférence le mélange d'anhydride maléigué et d'anhydrique acétique; à une température allant de -10°C à 80°C dans de solvants aliphatiques halogénés ou des esters C₂-C₄ d'acides aliphatiques C₂-C₄,
c) hydrolyse dans un solvant alcoolisé ou des éthers miscibles dans l'eau, avec un catalyseur basique, à une température de 10°C à 55°C, de l'ester de formule générale (IV), obtenu selon l'étape b): 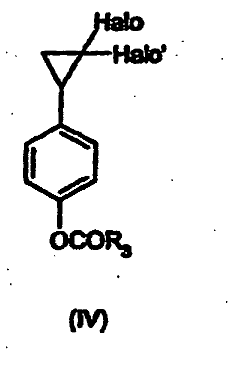 ou Halo, Halo' et R³ ont les significations ci-dessus, pour donner le phénol 4-dihalocyclopropyl- substitué correspondant;
d) réaction du phénol 4-dihalocyclopropryl-substitué, obtenu selon l'étape c), avec un α-haloester de la formule générale (V) : (R¹) (R²) CX'COOAlc, ou R¹, R² ont les significations ci-dessus, Alc est un groupe alkyle C₁-C₅ linéaire ou ramifié et X' est un halogène, dans un solvant alcoolisé avec catalyse basique, pour donner le composé de formule (VI) : e) saponification des esters de formule générale (VI), saponification des esters de formule générale (VI), 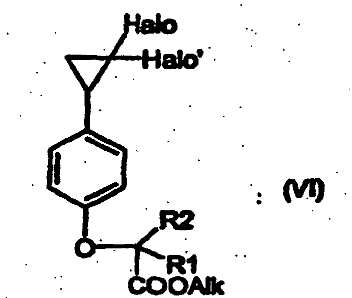
e) saponification des esters de formule générale (VI), obtenus selon l'étape d), avec des hydroxydes de métaux alcalins et acidification du produit préparé par saponification avec des acides pour donner le composé de formule générale (I),
**caractérisé en ce que**:
i) dans l'hydrosyle du composé de formule (IV), étape c), la base est une base inorganique sélectionnée dans le groupe consistant en carbonates alcalins, borates dibasiques alcalins/alcalino-terreux, phosphates dibasiques alcalins/alcalino-terreux ou leurs mélanges et
ii) les étapes c), d) et e), ont lieu dans le même récipient réactionnel sans isolement des intermédiaires.

2. Procédé selon la revendication 1 comprenant de plus l'étape f) pour la purification par cristallisation du composé de formule générale (I) par solubilisation dans des solvants sélectionnés dans le groupe consistant en hydrocarbures aromatiques ou aliphatiques C₆-C₁₀ et leurs mélanges.

3. Procédé selon la revendication 2 où les solvants sont un mélange de toluène et de n-hexane.

4. Procédé selon la revendication 3 où les solvants sont à un rapport égal à 1:4 en volume.

5. Procédé selon la revendication 1 où dans la réaction de Friedel-Crafts, étape a), l'halogénure d'acyle et le chlorure d'acétyle et le catalyseur, l'acide de Lewis, est sélectionné dans le groupe consistant en chlorure ferrique , FeCl₃, chlorure de zinc ZnCl₂, chlorure d'étain SnCl₂, chlorure d'aluminium AlCl₃ ou zéolites.

6. Procédé selon la revendication 5, où l'halogénure d'acyle est le chlorure d'acétyle et l'acide de Lewis et le chlorure d'aluminium AlCl₃.

7. Procédé selon la revendication 1 où la réaction de Friedel-Crafts, étape a) est effectuée dans le chlorure de méthylène.

8. Procédé selon la revendication 1 où la réaction de Friedel-Crafts est effectuée à une température allant de -5°C à 30°C, de préférence de 0°C à 20°C.

9. Procédé selon la revendication 1 où la réaction de Friedel-Crafts, étape a) est effectuée sur des composés de la formule générale (II), où Halo et Halo' sont sélectionnés dans le groupe consistant en chlore et brome, facultativement chlore.

10. Procédé selon la revendication 1, où la réaction de Baeyer-Villiger, étape b), est effectuée dans le chlorure de méthylène.

11. Procédé selon la revendication 1, où la réaction de Baeyer-Villiger, étape b), est effectuée à une température comprise entre -5°C et 60°C, facultativement entre 0 et 50°C.

12. Procédé selon la revendication 1 où, dans l'hydrolyse du composé de formule (IV), étape c), le solvant alcoolisé est sélectionné dans le groupe consistant en alcools aliphatiques C₁-C₄ linéaires ou ramifiés ou leurs mélanges.

13. Procédé selon la revendication 12 où le solvant alcoolisé est sélectionné dans le groupe consistant en méthanol, éthanol, n-propanol, isopropanol ou leurs mélanges.

14. Procédé selon la revendication 13, où le solvant alcoolisé est méthanol et/ou n-propanol.

15. Procédé selon la revendication 1, où dans l'hydrolyse du composé de formule (IV), étape c), le solvant est un éther miscible dans l'eau.

16. Procédé selon la revendication 15, où l'éther miscible dans l'eau est sélectionné dans le groupe consistant en tétrahydrofuranne (THF), dioxane, éthylène glycol monométhyl éther (METHYLCELLOSOLVE®), éthylène glycol monoéthyl éther (ETHYLCELLOSOLVE®).

17. Procédé selon la revendication 1 où la base inorganique est sélectionnée dans le groupe consistant en carbonate de sodium (Na₂CO₃), carbonate de potassium (K₂CO₃), ou leurs mélanges.

18. Procédé selon la revendication 17 où la base inorganique est du carbonate de potassium.

19. Procédé selon la revendication 1 où l'hydrolyse, étape c), est effectuée à une température allant de 20°C à 45°C, facultativement de 25 à 40°C.

20. Procédé selon la revendication 1 où dans l'α-haloester utilisé dans l'étape de réaction d), les groupes alkyles R¹ et R², qui sont identiques ou différents, sont sélectionnés dans le groupe consistant en méthyle, éthyle, n-propyle et isopropyle.

21. Procédé selon la revendication 1 où dans l'a-haloester utilisé dans l'étape de réaction d), le substituant Alc est sélectionné dans le groupe consistant en méthyle, éthyle, n-propyle, isopropyle.

22. Procédé selon la revendication 21 où le substituant Alk est un méthyle.

23. Procédé selon la revendication 1 où dans l'α-haloester utilisé, à l'étape de réaction d)X' est sélectionné parmi chlore et brome.

24. Procédé selon la revendication 1 où à l'étape d), le solvant alcoolisé est sélectionné dans le groupe consistant en alcools aliphatiques, linéaires où ramifiés C₁-C₄ ou leurs mélanges.

25. Procédé selon la revendication 24 où le solvant alcoolisé est sélectionné dans le groupe consistant en méthanol, éthanol, n-propanol, isopropanol, ou leurs mélanges.

26. Procédé selon la revendication 1, où la base à l'étape d) est une base inorganique sélectionnée dans le groupe consistant en carbonates alcalins ou leurs mélanges, ou une base organique sélectionnée dans le groupe consistant en alcoolates alcalins C₁-C₄ ou leurs mélanges.

27. Procédé selon la revendication 26, où la base inorganique est sélectionnée dans le groupe consistant en carbonate de sodium (Na₂CO₃), carbonate de potassium (K₂CO₃) ou leurs mélanges.

28. Procédé selon la revendication 27, où la base inorganique est du carbonate de potassium.

29. Procédé selon la revendication 26, où la base organique est sélectionnée dans le groupe consistant en méthylate de sodium (CH₃ONa) et éthylate de sodium (C₂H₅ONa) ou leurs mélanges.

30. Procédé selon la revendication 29, où la base organique est du méthylate de sodium.

31. Procédé selon la revendication 1, où à l'étape d), le solvant alcoolisé est n-propanol et la base est carbonate de potassium.

32. Procédé selon la revendication 1, où à l'étape d), le solvant alcoolisé est isopropanol et la base est une solution de méthylate de sodium dans le méthanol.

33. Procédé selon la revendication 1, où la saponification, étape e) se produit avec des solutions aqueuses de carbonate de sodium ou carbonate de potassium et l'acidification se produit avec des acides sélectionnés dans le groupe consistant en acide sulfurique, phosphorique ou acétique.

34. Procédé selon l'une quelconque des revendications 1 et 2, pour la préparation d'acides 2-[4-(2,2-dihalocyclopropyl)phénoxy]-2-alkylpropanoiques de formule (VII) 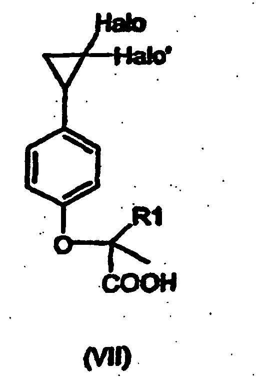 où, dans l'α-haloester de formule générale (V) utilisé à l'étape de réaction d), l'un des deux groupes alkyles, R¹ ou R² est un méthyle.

35. Procédé selon l'une quelconque des revendications 1 et 2 pour la préparation du composé acide 2-[4-(2,2-dichlorocyclopropyl)phénoxy]-2-méthylpropanoïque de formule (VIII) 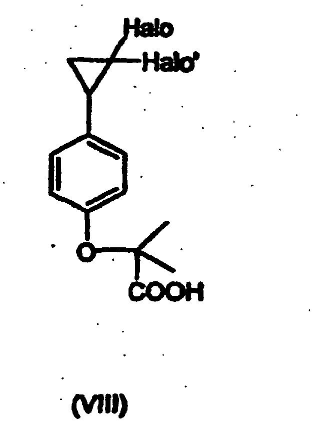 où le composé de formule générale (II) est le 2,2-dichlorocyclopropyl benzène et l'α-haloester de formule générale (V) est l'ester alkylique C₁-C₅ linéaire ou ramifié de l'acide α-bromoisobutyrique.

36. Procédé selon la revendication 35 où l'ester alkylique de l'acide α-bromoisobutyrique est sélectionné dans le groupe consistant en méthyl, éthyl, n-propyl, et isopropyl ester de l'acide α-bromoisobutyrique.

37. Procédé selon la revendication 36 où l'ester de l'acide α-bromoisobutyrique est l'α-bromoisobutyrate de méthyle.
